(19) Europäisches Patentamt
European Patent Office
Office européen des brevets

(11) EP 4 332 080 A1

(12) EUROPEAN PATENT APPLICATION
published in accordance with Art. 153(4) EPC

(43) Date of publication:
**06.03.2024 Bulletin 2024/10**

(21) Application number: **22795512.7**

(22) Date of filing: **31.03.2022**

(51) International Patent Classification (IPC):
***C07C 39/06*** (2006.01) ***C08F 110/14*** (2006.01)
***C08F 2/40*** (2006.01) ***C07C 69/24*** (2006.01)
***C07C 69/54*** (2006.01)

(52) Cooperative Patent Classification (CPC):
(C-Sets available)
**C07C 67/62; C08F 2/40; C08F 110/14** (Cont.)

(86) International application number:
**PCT/JP2022/016404**

(87) International publication number:
**WO 2022/230596 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2021 JP 2021075625**

(71) Applicant: **Mitsubishi Chemical Corporation**
**Chiyoda-ku**
**Tokyo 100-8251 (JP)**

(72) Inventors:
- **KURIHARA, Yu**
  **Tokyo 100-8251 (JP)**
- **KATO, Yuki**
  **Tokyo 100-8251 (JP)**
- **SUZUKI, Tatsuya**
  **Tokyo 100-8251 (JP)**
- **NINOMIYA, Wataru**
  **Tokyo 100-8251 (JP)**

(74) Representative: **Hoffmann Eitle**
**Patent- und Rechtsanwälte PartmbB**
**Arabellastraße 30**
**81925 München (DE)**

(54) **METHYL METHACRYLATE-CONTAINING COMPOSITION**

(57) An object of the present invention is to provide a methyl methacrylate-containing composition having high storage and heat stability. This can be solved with a methyl methacrylate-containing composition containing methyl methacrylate, an ester compound having an alpha-hydrogen represented by Formula (1) (component A), and a polymerization inhibitor (component B), in which the concentration of methyl methacrylate is from 99% by mass to 99.99% by mass.

EP 4 332 080 A1

(52) Cooperative Patent Classification (CPC): (Cont.)

C-Sets
**C07C 67/62, C07C 69/54**

## Description

TECHNICAL FIELD

The present invention relates to a methyl methacrylate-containing composition.

BACKGROUND ART

Methyl methacrylate (hereinafter also referred to as "MMA") is known to be an extremely useful substance used as a raw material of various applications and types of polymers. For example, polymethyl methacrylate, a homopolymer of methyl methacrylate, is used in signboards, lighting equipment, automotive parts, construction-related materials, light guiding panels for flat displays, light diffusion plates, and the like, taking advantage of its excellent transparency, weather resistance, and other properties. In addition, copolymers of methyl methacrylate and other monomers are used in paints, adhesives, resin modifier, artificial marble, latices for paper, and the like. Various methods have been developed for industrially producing methyl methacrylate, and for example, the acetone cyanohydrin (ACH) method, the new acetone cyanohydrin (new ACH) method, the C4 direct oxidation method, the direct methyl esterification method, the ethylene method, and the new ethylene method are known (Non-Patent Document 1). In these production methods, methyl methacrylate of a quality suitable for the intended use is obtained by performing purification such as distillation to remove unreacted raw materials and by-products contained in the produced methyl methacrylate.

Since methyl methacrylate has a tendency to polymerize, it is known that the quality of methyl methacrylate is maintained by adding a polymerization inhibitor when producing methyl methacrylate or storing produced methyl methacrylate (Non-Patent Document 2). For example, Patent Document 1 describes that methyl ether of hydroquinone (MEHQ) is particularly preferable among various polymerization inhibitors. Patent Document 2 describes that N,N'-dialkyl-p-phenylenediamine and N-oxyl are preferable among various polymerization inhibitors. Patent Document 3 describes distillation of methyl methacrylate in the presence of a phenol polymerization inhibitor. Patent Document 4 describes use of a diphenylamine derivative as a polymerization inhibitor. Patent Document 5 describes use of a benzene triamine derivative as a polymerization inhibitor.

PRIOR ART DOCUMENTS

PATENT DOCUMENTS

Patent Document 1: JP 2004-155757 A
Patent Document 2: JP 2005-502695 A
Patent Document 3: JP H10-504553 A
Patent Document 4: JP 2002-533309 A
Patent Document 5: JP 2002-513034 A

NON-PATENT DOCUMENTS

Non-Patent Document 1: Toru Kuroda, "Development of Catalyst for Producing Methyl Methacrylate", Catalysts & Catalysis, 45 (5), 366-371, 2003, Catalysis Society of Japan

Non-Patent Document 2: Takayuki Otsu, "On the Functions of Polymerization Inhibitors", Synthetic Organic Chemistry, 33 (8), 634-640, 1975, The Society of Synthetic Organic Chemistry, Japan

SUMMARY OF THE INVENTION

PROBLEMS TO BE SOLVED BY THE INVENTION

However, even in cases where polymerization inhibitors as described above are added, the quality of methyl methacrylate may be deteriorated during storage. In view of the above circumstances, the purpose of the present invention is to provide a methyl methacrylate-containing composition with high quality stability during storage.

MEANS FOR SOLVING THE PROBLEMS

In order to achieve the above purposes, the present inventors have intensively studied. As a result, they found

that, in methyl methacrylate with the quality deteriorated during storage, the concentration of methyl methacrylate is reduced, and methyl methacrylate dimers and methyl pyruvate are produced. The presence of methyl methacrylate dimers in methyl methacrylate may change the structure of methyl methacrylate polymer obtained by polymerization to adversely affect its physical properties. Furthermore, the presence of methyl pyruvate in methyl methacrylate causes coloration of methyl methacrylate polymer obtained by polymerization. The present inventors have found that inclusion of an ester compound having an alpha-hydrogen represented by a specific structural formula, and a specific polymerization inhibitor in a methyl methacrylate-containing composition results in improved quality stability during storage, and reduced production of methyl methacrylate dimers and methyl pyruvate, thereby completing the present invention.

Accordingly, the present invention provides the following [1] to [18]:

[1]: A methyl methacrylate-containing composition, comprising:

methyl methacrylate;
an ester compound having an alpha-hydrogen represented by the following Formula (1) (component A); and
a polymerization inhibitor (component B),
wherein the concentration of methyl methacrylate is from 99 to 99.99% by mass,

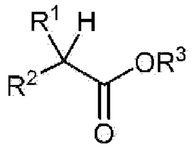 (1)

wherein, in Formula (1) above,
$R^1$ and $R^2$ each independently represent a hydrogen atom, an alkyl group, an alkenyl group, a hydroxy group, an alkoxy group, an amino group, a monovalent group containing a carbonyl group, or an alkylthio group,
$R^3$ represents an alkyl group or an aryl group, and
$R^1$ and $R^2$, $R^2$ and $R^3$, and $R^3$ and $R^1$ each may be linked to each other to form a ring;

[2]: The methyl methacrylate-containing composition according to [1], wherein when the concentration of the component A is MA (umol/L) and the concentration of the component B is MB (μmol/L), MB/MA is from 0.005 to 7;
[3]: The methyl methacrylate-containing composition according to [1] or [2], wherein the MB/MA is from 0.05 to 5;
[4]: The methyl methacrylate-containing composition according to any of [1] to [3], wherein the MA is from 1 to 50,000 (pmol/L);
[5]: The methyl methacrylate-containing composition according to any of [1] to [4], wherein the MA is from 10 to 30,000 (pmol/L);
[6]: The methyl methacrylate-containing composition according to any of [1] to [5], wherein the MB is from 1 to 5,000 (μmol/L);
[7]: The methyl methacrylate-containing composition according to any of [1] to [6], wherein the MB is from 10 to 1,000 (pmol/L);
[8]: The methyl methacrylate-containing composition according to any of [1] to [7], wherein the molecular weight of the component A is 1,000 or less;
[9]: The methyl methacrylate-containing composition according to any of [1] to [8], wherein the component B is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound;
[10]: The methyl methacrylate-containing composition according to any of [1] to [9], wherein the component B is at least one polymerization inhibitor selected from the group consisting of a phenol compound and a sulfur-containing compound;
[11]: The methyl methacrylate-containing composition according to any of [1] to [10], wherein the component B is at least one polymerization inhibitor selected from the group consisting of hydroquinone, 4-methoxyphenol, 2,4-dimethyl-6-t-butylphenol, 2,6-di-t-butyl-4-methylphenol, and phenothiazine;
[12]: The methyl methacrylate-containing composition according to any of [1] to [11], wherein the concentration of methyl methacrylate is from 99.8 to 99.99% by mass;
[13]: The methyl methacrylate-containing composition according to any of [1] to [12], wherein the composition does not contain diacetyl, or the concentration of diacetyl contained is 55 (μmol/L) or less;
[14]: The methyl methacrylate-containing composition according to any of [1] to [13], wherein in Formula (1), $R^1$ and

$R^2$ are each independently a hydrogen atom, a $C_{1-5}$ alkyl group, a hydroxy group, a $C_{1-6}$ alkoxy group, an amino group, a monovalent group containing a carbonyl group, or a $C_{1-5}$ alkylthio group; and $R^3$ is a $C_{1-5}$ alkyl group or a $C_{5-12}$ aryl group;

[15]: The methyl methacrylate-containing composition according to any of [1] to [14], wherein in Formula (1), $R^1$ and $R^2$ are each independently a hydrogen atom or a $C_{1-5}$ alkyl group; and $R^3$ is a $C_{1-5}$ alkyl group;

[16]: The methyl methacrylate-containing composition according to any of [1] to [15], wherein in Formula (1), $R^1$ and $R^2$ are each independently a hydrogen atom, a methyl group, an ethyl group, an n-propyl group, or an isopropyl group; and $R^3$ is a methyl group;

[17]: A method of producing a methyl methacrylate polymer, comprising a step of polymerization of a polymeric composition comprising a methyl methacrylate-containing composition according to any of [1] to [16]; and

[18]: The method of producing a methyl methacrylate polymer according to [17], wherein the polymeric composition comprises a monomer that can be copolymerized with methyl methacrylate.

## EFFECT OF THE INVENTION

According to the present invention, a methyl methacrylate-containing composition having high quality stability with reduced production of methyl methacrylate dimer and methyl pyruvate during storage can be provided.

## DETAILED DESCRIPTION OF THE INVENTION

Embodiments according to the present invention will be described below, but the present invention is not limited to them.

As used herein, any numerical value range indicated by the term "to" means any range including the numerical values described before and after the term "to" as the lower and upper limit values, respectively, and "A to B" means A or more and B or less.

### [Methyl Methacrylate-containing Composition]

A methyl methacrylate-containing composition according to the present invention comprises methyl methacrylate, an ester compound having an alpha-hydrogen represented by the following Formula (1) (component A), and a polymerization inhibitor (component B), wherein the concentration of methyl methacrylate is from 99 to 99.99% by mass.

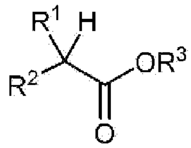 (1)

In Formula (1) above, $R^1$ and $R^2$ each independently represent a hydrogen atom, an alkyl group, an alkenyl group, a hydroxy group, an alkoxy group, an amino group, a monovalent group containing a carbonyl group, or an alkylthio group. $R^3$ represents an alkyl group or an aryl group. $R^1$ and $R^2$, $R^2$ and $R^3$, and $R^3$ and $R^1$ each may be linked to each other to form a ring.

The methyl methacrylate-containing composition may also contain other compound (component C) or water as long as the composition satisfies a concentration of methyl methacrylate of from 99 to 99.99% by mass. Each item will be described in detail.

### (Component A)

The methyl methacrylate-containing composition according to the present invention comprises an ester compound (component A) having an alpha-hydrogen represented by Formula (1) described above. The term "alpha-hydrogen" represents a hydrogen atom bound to a carbon atom next to a carbon atom of a carbonyl group. Coexistence of the component A and a component B described later enables reducing the production of methyl methacrylate dimers and methyl pyruvate. The reason for this is presumed as follows.

The component B described later traps radicals generated in methyl methacrylate, thereby reducing the polymerization reaction of methyl methacrylate by radical polymerization. However, the dimerization reaction of methyl methacrylate also proceeds by anionic polymerization under a basic condition. An ester compound having an alpha-hydrogen is a weak acid and can trap an anion that causes anionic polymerization. Thus, the component A can reduce the dimerization reaction of methyl methacrylate by anionic polymerization. On the other hand, methyl pyruvate is

produced by oxidation of methyl methacrylate with hydroxy radicals and oxygen molecules. The component B can trap hydroxy radicals, while the component A can trap a radical intermediate produced by the reaction of a hydroxy radical and methyl methacrylate and convert the intermediate back to methyl methacrylate. Thus, it is considered that coexistence of the component A and the component B enables efficiently reducing the production of methyl pyruvate.

The molecular weight of the component A is preferably 1,000 or less. When the molecular weight is 1,000 or less, the number of alpha-hydrogens per unit mass in the component A can be increased, so that the effect of the present invention can be obtained with less mass. The molecular weight of the component A is more preferably 800 or less, still more preferably 600 or less, and particularly preferably 400 or less.

$R^1$ and $R^2$ in Formula (1) described above each independently represent a hydrogen atom, an alkyl group, an alkenyl group, a hydroxy group, an alkoxy group, an amino group, a monovalent group containing a carbonyl group, or an alkylthio group. $R^1$ and $R^2$ may be the same or different. $R^3$ in Formula (1) described above represents an alkyl group, an alkenyl group, or an aryl group. $R^3$ and $R^1$, and $R^3$ and $R^2$ may be the same or different. $R^1$ and $R^2$, $R^2$ and $R^3$, and $R^3$ and $R^1$ each may be linked to each other to form a ring.

In general, an alpha-hydrogen in an ester compound is reactive with an anion or a radical, and may have reduced reactivity depending on the type of the substituent. In cases where $R^1$, $R^2$ and $R^3$ satisfy the conditions described above, the reactivity of the alpha-hydrogen of the component A with an anion or a radical is maintained, so that the effect of the present invention can be obtained. $R^1$ and $R^2$ each are preferably a hydrogen atom, a $C_{1-5}$ alkyl group, a hydroxy group, a $C_{1-6}$ alkoxy group, an amino group, a monovalent group containing a carbonyl group, or a $C_{1-5}$ alkylthio group. They are highly stable substituents, and thus can prevent the component A from being changed into other compounds during storage. In addition, they also have low electron-donating properties, so that the acidic properties of the alpha-hydrogen of the component A are increased. $R^1$ and $R^2$ each are more preferably a hydrogen atom or a $C_{1-5}$ alkyl group, and still more preferably a hydrogen atom, a methyl group, an ethyl group, an n-propyl group, or an isopropyl group. $R^3$ is more preferably a $C_{1-5}$ alkyl group or a $C_{5-12}$ aryl group. They are highly stable substituents, and thus can prevent the component A from being changed into other compounds during storage. $R^3$ is more preferably a $C_{1-5}$ alkyl group, and still more preferably a methyl group.

The alkyl group is a chain (straight-chain or branched-chain) alkyl group or a cyclic alkyl group. In cases where the alkyl group is a chain alkyl group, it is preferably a $C_{1-20}$ chain alkyl group, more preferably a $C_{1-10}$ chain alkyl group, and still more preferably a $C_{1-5}$ chain alkyl group. Examples of the chain alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, and an isopropyl group are preferable. In cases where the alkyl group is a cyclic alkyl group, it is preferably a $C_{3-20}$ cyclic alkyl group, more preferably a $C_{4-10}$ cyclic alkyl group, and still more preferably a $C_{5-7}$ cyclic alkyl group. Examples of the cyclic alkyl group include a cyclopentyl group, a cyclohexyl group, and a cyclooctyl group.

The alkenyl group is a chain (straight-chain or branched-chain) alkenyl group or a cyclic alkenyl group. In cases where the alkenyl group is a chain alkenyl group, it is preferably a $C_{2-20}$ chain alkenyl group, more preferably a $C_{2-10}$ chain alkenyl group, and still more preferably a $C_{2-5}$ chain alkenyl group. Examples of the chain alkenyl group include a vinyl group, a 1-propenyl group, an isopropenyl group, a 2-butenyl group, a 1,3-butadienyl group, a 2-pentenyl group, and 2-hexenyl group. In cases where the alkenyl group is a cyclic alkenyl group, it is preferably a $C_{3-20}$ cyclic alkenyl group, more preferably a $C_{4-10}$ cyclic alkenyl group, and still more preferably a $C_{5-7}$ cyclic alkenyl group. Examples of the cyclic alkenyl group include a cyclopentenyl group and a cyclohexenyl group.

The alkoxy group is preferably a $C_{1-20}$ alkoxy group, more preferably a $C_{1-10}$ alkoxy group, and still more preferably a $C_{1-6}$ alkoxy group. Examples of the alkoxy group include a methoxy group, an ethoxy group, a butoxy group, and a phenoxy group.

The amino group includes an amino group without a substituent on the nitrogen atom ($-NH_2$), and an amino group in which some or all of the hydrogen atoms bound to the nitrogen atom are substituted with carbon atoms. The number of carbon atoms in an amino group substituted with carbon atoms is preferably from 1 to 20, more preferably from 1 to 10, and still more preferably from 1 to 5. Examples of the amino group include a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group containing a carbonyl group include a formyl group, an acyl group, a carboxy group, an amide group, an alkoxy carbonyl group, a thiocarboxy group, and a thioester group.

The acyl group is a substituent in which a carbonyl group is linked with an alkyl group, an alkenyl group, or an aryl group. The total number of carbon atoms derived from a carbonyl group (one) and derived from an alkyl group, alkenyl group, or an aryl group is preferably from 2 to 20, more preferably from 2 to 10, and still more preferably from 2 to 6. Examples of the acyl group include an acetyl group, a propionyl group, a butylcarbonyl group, a vinylcarbonyl group, and a benzoyl group.

The amide group includes an amide group without a substituent on the nitrogen atom ($-CONH_2$), and an amide

group in which some or all of the hydrogen atoms bound to the nitrogen atom are substituted with carbon atoms. The number of carbon atoms in the amide group, which is the total number of carbon atoms derived from a carbonyl group (one) and the number of carbon atoms substituted on the nitrogen atom, is preferably from 1 to 20, more preferably from 1 to 10, and still more preferably from 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxy carbonyl group is a substituent in which a carbonyl group and an alkoxy group are linked, and also referred to as an ester group. The total number of carbon atoms derived from a carbonyl group (one) and derived from an alkoxy group is preferably from 2 to 20, more preferably from 2 to 10, and still more preferably from 2 to 6. Examples of the alkoxy carbonyl group include a methoxycarbonyl group, an ethoxycarbonyl group, a butoxycarbonyl group, and a phenoxycarbonyl group.

The thioester group is a substituent in which a carbonyl group and an alkylthio group or an arylthio group are linked. The total number of carbon atoms derived from a carbonyl group (one) and derived from an alkylthio group or an arylthio group is preferably from 2 to 20, more preferably from 2 to 10, and still more preferably from 2 to 6. Examples of the thioester group include a methylthiocarbonyl group, an ethylthiocarbonyl group, a butylthiocarbonyl group, and a phenylthiocarbonyl group.

The monovalent group containing a carbonyl group may be a substituent in which one or more hydrogen(s) in an alkyl group is/are substituted with a carbonyl group(s). Examples of such a substituent include a 2-acetoxyethyl group, a 2-acetoethyl group, and a 2-(acetoacetoxy)ethyl group.

The alkylthio group is preferably a $C_{1-20}$ alkylthio group, more preferably a $C_{1-10}$ alkylthio group, and still more preferably a $C_{1-5}$ alkylthio group. Examples of the alkylthio group include a methylthio group, an ethylthio group, a propylthio group, and an isopropylthio group.

The aryl group is preferably a $C_{5-20}$ aryl group, and more preferably a $C_{5-12}$ aryl group. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a thiophenyl group, a triazolyl group, a tetraazolyl group, a pyridyl group, a pyrazinyl group, a pyrimidinyl group, a pyridazinyl group, a pyrazolyl group, a pyrrolyl group, a pyranyl group, a furyl group, a furazanyl group, an imidazolidinyl group, an isoquinolyl group, an isoindolyl group, an indolyl group, a quinolyl group, a pyridothiazolyl group, a benzimidazolyl group, a benzooxazolyl group, a benzothiazolyl group, a benzotriazolyl group, a benzofuranyl group, an imidazopyridinyl group, a triazopyridinyl group, and a purinyl group.

$R^1$ and $R^2$, $R^2$ and $R^3$, and $R^3$ and $R^1$ each may be linked to each other to form a ring. Examples of the compound in which $R^1$ and $R^2$ are linked to form a ring, and the compound in which $R^3$ and $R^1$ are linked to form a ring include methyl cyclohexanecarboxylate, and methyl cyclopentanecarboxylate. Examples of the compound in which $R^2$ and $R^3$ are linked to form a ring include $\alpha$-methyl-$\delta$-valerolactone, and $\alpha$-methyl-$\gamma$-butyrolactone.

Among compounds that satisfy the conditions described above, the component A is preferably methyl isobutyrate, methyl propionate, isobutyl isobutyrate, methyl isovalerate, methyl 2-methylbutyrate, isoamyl isobutyrate, methyl lactate, methyl 2-methoxypropionate, N,N-dimethylglycine methyl, dimethyl malonate, methyl (methylthio)acetate, methyl 3-butenoate, methyl (R)-(-)-3-hydroxyisobutyrate, methyl acetate, ethyl acetate, phenyl acetate, ethyl propionate, ethyl isobutyrate, phenyl isobutyrate, methyl butyrate, methyl cyclohexanecarboxylate, methyl cyclopentanecarboxylate, $\alpha$-methyl-$\delta$-valerolactone, or $\alpha$-methyl-$\gamma$-butyrolactone; more preferably methyl isobutyrate, methyl propionate, isobutyl isobutyrate, methyl isovalerate, methyl 2-methylbutyrate, isoamyl isobutyrate, methyl lactate, N,N-dimethylglycine methyl, dimethyl malonate, methyl (methylthio)acetate, methyl 3-butenoate, methyl (R)-(-)-3-hydroxyisobutyrate, or methyl cyclohexanecarboxylate; and still more preferably methyl isobutyrate, methyl propionate, isobutyl isobutyrate, or methyl 2-methylbutyrate, from the viewpoint of quality stability of methyl methacrylate-containing composition during storage.

One or two or more component(s) A may be contained.

(Component B)

The methyl methacrylate-containing composition according to the present invention comprises a polymerization inhibitor (component B). As used herein, the term "polymerization inhibitor" means a compound having a function to inhibit the polymerization reaction of methyl methacrylate. Examples of the polymerization inhibitor include a phenol compound, a quinone compounds, nitrobenzene compound, an N-oxyl compound, an amine compound, a phosphorus-containing compound, a sulfur-containing compound, an iron-containing compound, a copper-containing compound, and a manganese-containing compound. When the component B is included, the polymerization reaction of methyl methacrylate via a radical polymerization mechanism can be repressed. As described above, when a component A and the component B coexist, the production of methyl pyruvate caused by oxidation of methyl methacrylate can be efficiently

reduced.

Examples of the polymerization inhibitor that is a phenol compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylene-bis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzoate.

Examples of the tocopherol include $\alpha$-tocopherol, and 2,3-dihydro-2,2-dimethyl-7-hydroxybenzofuran.

Examples of the polymerization inhibitor that is a quinone compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor that is a nitrobenzene compound include nitrobenzene, o-dinitrobenzene, m-dinitrobenzene, p-dinitrobenzene, 2,4-dinitrotoluene, dinitrodurene, and 2,2-diphenyl-1-picrylhydrazyl.

Examples of the polymerization inhibitor that is an N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethanoloxy-2,2,6,6-tetramethyl-piperidine-N-oxyl, 4-benzoyloxy-2,2,6,6-tetramethylpiperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butylnitroxide, and di-tert-amylnitroxide.

Examples of the polymerization inhibitor that is an amine compound include N,N-diphenylamine, alkylated diphenylamine, 4,4'-dicumyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (the alkyl groups may be the same or different, and may each independently comprise 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-$\alpha$-naphthylamine, N-phenyl-$\beta$-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor that is a phosphorus-containing compound include triphenylphosphine, triphenyl phosphite, triethyl phosphite, tris(isodecyl) phosphite, tris(tridecyl) phosphite, phenyl diisooctyl phosphite, phenyl diisodecyl phosphite, phenyl di(tridecyl) phosphite, diphenyl isooctyl phosphite, diphenyl isodecyl phosphite, diphenyl tridecyl phosphite, phosphonic acid [1,1-diphenyl-4,4'-diylbistetrakis-2,4-bis(1,1-dimethylethyl)phenyl] ester, triphenyl phosphite, tris(nonylphenyl) phosphite, 4,4'-isopropylidenediphenol alkyl phosphite, tris(2,4-di-tert-butylphenyl) phosphite, tris(biphenyl) phosphite, distearyl pentaerythritol diphosphite, di(2,4-di-tert-butylphenyl) pentaerythritol diphosphate, di(nonylphenyl) pentaerythritol diphosphite, phenyl bisphenol-A pentaerythritol diphosphite, tetra(tridecyl)-4,4'-butylidenebis(3-methyl-6-tert-butylphenol)diphosphite, hexa(tridecyl)-1,1,3-tris(2-methyl-4-hydroxy-5-tert-butylphenyl) butanetriphosphite, 3,5-di-tert-butyl-4-hydroxybenzyl phosphate diethyl ester, sodium-bis(4-tert-butylphenyl) phosphate, sodium-2,2'-methylene -bis(4,6-di-tert-butylphenyl)phosphate, and 1,3-bis(diphenoxyphosphonyloxy)benzene.

Examples of the polymerization inhibitor that is a sulfur-containing compound include diphenyl sulfide, phenothiazine, 3-oxophenothiazine, 5-oxophenothiazine, phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor that is an iron-containing compound include iron (III) chloride.

Examples of the polymerization inhibitor that is a copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor that is a manganese-containing compound include manganese dialkyl dithiocarbamate (the alkyl groups are each any of a methyl group, an ethyl group, a propyl group, and a butyl group, and may be the same or different), manganese diphenyl dithiocarbamate, manganese formate, manganese acetate, manganese octanoate, manganese naphthenate, manganese permanganate, and ethylenediaminetetraacetic acid manganese salt.

Among those described above, preferred is/are at least one selected from the group consisting of the phenol compound and the sulfur-containing compound, and preferred is hydroquinone, 4-methoxyphenol, 2,4-dimethyl-6-t-butylphenol, 2,6-di-t-butyl-4-methylphenol, or phenothiazine, from the viewpoint of quality stability of the methyl methacrylate-containing composition during storage.

One or two or more component(s) B may be contained.

In cases where the methyl methacrylate-containing composition comprises a compound that corresponds to both components A and B, the compound is considered as a component B. Thus, the methyl methacrylate-containing composition must comprise a component A other than the compound. In cases where two or more compounds that correspond to both components A and B are contained, the compound with the highest concentration in the methyl methacrylate composition is considered as a component B, and the other compound(s) is/are considered as a component(s) A.

(Concentrations of Component A and Component B)

When the concentration of a component A is MA (umol/L) and the concentration of a component B is MB (umol/L), then MB/MA is preferably from 0.005 to 7. More preferably, the lower limit of MB/MA is 0.05 or more, and the upper limit is 5 or less, from the viewpoint of efficiency in reducing the production of methyl pyruvate.

MA is preferably from 1 to 50,000 μmol/L. When MA is 1 μmol/L or more, the effect of reducing the production of methyl methacrylate dimer and methyl pyruvate can be sufficiently obtained. When MA is 50,000 μmol/L or less, the impurity content after production of methyl methacrylate polymer by polymerization of the methyl methacrylate-containing composition according to the present invention is reduced, so that adverse effects on physical properties of the polymer can be prevented. The lower limit of MA is more preferably 10 μmol/L or more, and still more preferably 50 μmol/L or more. The upper limit of MA is more preferably 30,000 μmol/L or less, and still more preferably 10,000 μmol/L or less.

MB is preferably from 1 to 5,000 μmol/L. When MB is 1 μmol/L or more, the effect of reducing the production of methyl methacrylate dimer and methyl pyruvate can be sufficiently obtained. When MB is 5,000 μmol/L or less, the impurity content after production of methyl methacrylate polymer by polymerization of the methyl methacrylate-containing composition according to the present invention is reduced, so that adverse effects on physical properties of the polymer can be prevented. The lower limit of MB is more preferably 10 μmol/L or more, and still more preferably 40 μmol/L or more. The upper limit of MB is more preferably 1,000 μmol/L or less, and still more preferably 600 μmol/L or less.

(Concentration of Methyl Methacrylate)

The concentration of methyl methacrylate in the methyl methacrylate-containing composition according to the present invention is from 99 to 99.99% by mass. When the concentration of methyl methacrylate is 99% by mass or more, the impurity content after production of methyl methacrylate polymer by polymerization of the methyl methacrylate-containing composition according to the present invention is reduced, so that adverse effects on physical properties of the polymer can be prevented. When the concentration of methyl methacrylate is 99.99% by mass or less, the purification cost can be reduced. The lower limit of the concentration of methyl methacrylate is preferably 99.8% by mass or more.

(Component C)

The methyl methacrylate-containing composition according to the present invention may contain other compound (component C) as long as the composition satisfies a concentration of methyl methacrylate of from 99 to 99.99% by mass. The component C includes, for example, impurities produced during the production of methyl methacrylate. For example, methyl methacrylate produced by C4 direct oxidation method may contain diacetyl as an impurity. From the viewpoint of reducing coloration of the methyl methacrylate-containing composition, the concentration of diacetyl is

preferably 55 (umol/L) or less, more preferably 20 (umol/L) or less, and still more preferably 10 (umol/L) or less.

(Analysis of Methyl Methacrylate-containing Composition)

Inclusion of a component A, a component B, or a component C by the methyl methacrylate-containing composition can be determined, for example, by GC-MS measurement. When the GC-MS chart of the methyl methacrylate-containing composition has a peak at the same retention time as a reference material for the component A, and when the m/z value detected in the mass spectrum of the peak corresponds to the exact mass of the component A, it can be determined that the methyl methacrylate-containing composition contains the component A. In cases where the reference material of the component A is not available, it can be determined that the peak is the component A peak when the mass spectrum peak pattern shown in the GC-MS chart of the methyl methacrylate-containing composition corresponds to the mass spectrum pattern of the component A recorded in the mass spectrum database. In other words, it can be determined that the methyl methacrylate-containing composition comprises the component A. Examples of the mass spectrum database include NIST20, NIST17, NIST14, and NIST14s. When detection by GC-MS measurement is impossible due to low volatility, LC-MS can be used for detection. Inclusion of a component B and a component C can also be determined by the same method.

The concentration of methyl methacrylate can be determined, for example, by performing GC-FID measurement of the methyl methacrylate-containing composition, quantifying methyl methacrylate using the area normalization method, and correcting the resulting value using the water concentration determined with a Karl-Fisher moisture meter. The concentration of the component A can be determined, for example, by performing GC measurement of the methyl methacrylate-containing composition and using the internal standard method. When the reference material of the component A is not available, and thus cannot be quantified by the internal standard method, GC-FID measurement for any organic compound having known concentration is performed, and then the concentration of the component A can be calculated using the following equation:

$$\text{Concentration of component A } (\mu\text{mol/L}) = \frac{N}{N_A} \times \frac{S_A}{S} \times \mathrm{M}$$

In the equation, N is the number of carbon atoms that any organic compound contains in one molecule; $N_A$ is the number of carbon atoms that the component A contains in one molecule; $S_A$ is the peak area of the component A, S is the peak area of any organic compound, and M is the concentration (μmol/L) of any organic component.

When quantification by GC measurement is impossible due to low volatility, a chromatography method such as LC can be used for quantification.

The concentrations of the component B and component C can be also determined by the same method as the component A described above.

Inclusion of water by the methyl methacrylate-containing composition and its concentration can be determined by the Karl-Fischer method.

[Method of Producing Methyl Methacrylate-containing Composition]

The method of producing the methyl methacrylate-containing composition according to the present invention may be a method in which a component A and a component B are added to methyl methacrylate. Methyl methacrylate to be used may be a commercially available product, or methyl methacrylate produced by known methods such as the acetone cyanohydrin (ACH) method, the new acetone cyanohydrin (new ACH) method, the C4 direct oxidation method, the direct methyl esterification method, the ethylene method, and the new ethylene method may be used. The component A and component B to be used may be commercially available products, or those synthesized by known methods may be used. When methyl methacrylate produced by a known method such as the acetone cyanohydrin (ACH) method, the new acetone cyanohydrin (new ACH) method, the C4 direct oxidation method, the direct methyl esterification method, the ethylene method, or the new ethylene method is used, the component A or the component B may be added to raw materials or in the course of the production process to produce the methyl methacrylate-containing composition. In cases where a component A or a component B is produced as a by-product during the methyl methacrylate production process, a part of the component A or the component B produced may be left for production of the methyl methacrylate-containing composition.

[Methods of Evaluating Storage Stability and Heat Stability]

The methyl methacrylate-containing composition according to the present invention has high quality stability

during storage. The method of evaluating the quality stability of the methyl methacrylate-containing composition during storage may be, for example, a method in which the methyl methacrylate-containing composition is actually stored for a long period, and the amounts of methyl methacrylate dimers and methyl pyruvate produced are determined. From the viewpoint of work simplicity, a method may be carried out in which the methyl methacrylate-containing composition is heated for a short time, and the amounts of methyl methacrylate dimers and methyl pyruvate produced are determined. In cases of heating for a short time, the heating temperature is preferably from 50 to 100°C, and the heating time period is preferably from 1 to 24 hours. In the present invention, the quality stability of the methyl methacrylate-containing composition during storage is evaluated based on the amounts of methyl methacrylate dimers and methyl pyruvate produced when the methyl methacrylate-containing composition is stored at 25°C for 14 days.

[Method of Producing Methyl Methacrylate Polymer]

The method of producing methyl methacrylate polymer according to the present invention comprises a step of polymerizing a polymeric composition comprising the methyl methacrylate-containing composition according to the present invention.

<Polymeric Composition>

The polymeric composition may comprise, as needed, a monomer that can be copolymerized with methyl methacrylate, and other additives.

(Monomer that can be Copolymerized with Methyl Methacrylate)

Examples of the monomer that can be copolymerized with methyl methacrylate include the following:

a methacrylate ester, such as ethyl methacrylate, isopropyl methacrylate, n-butyl methacrylate, isobutyl methacrylate, tert-butyl methacrylate, 2-ethylhexyl methacrylate, phenyl methacrylate, or benzyl methacrylate;
an acrylate ester, such as methyl acrylate, ethyl acrylate, n-butyl acrylate, isobutyl acrylate, tert-butyl acrylate, or 2-ethylhexyl acrylate;
an unsaturated carboxylic acid, such as acrylic acid, methacrylic acid, maleic acid, or itaconic acid;
an unsaturated carboxylic anhydride, such as maleic anhydride, or itaconic anhydride;
maleimide, such as N-phenylmaleimide, or N-cyclohexylmaleimide;
a vinyl monomer containing a hydroxy group, such as 2-hydroxyethyl acrylate, 2-hydroxyethyl methacrylate, or 2-hydroxypropyl methacrylate;
a vinyl ester, such as vinyl acetate, or vinyl benzoate;
vinyl chloride, vinylidene chloride, and derivatives thereof;
a vinyl monomer containing nitrogen, such as methacrylamide, or acrylonitrile;
a monomer containing an epoxy group, such as glycidyl acrylate, or glycidyl methacrylate;
an aromatic vinyl monomer, such as styrene, or alpha-methylstyrene;
alkane diol di(meth)acrylate, such as ethylene glycol di(meth)acrylate, 1,2-propylene glycol di(meth)acrylate, 1,3-butyleneglycol di(meth)acrylate, or 1,6-hexanediol di(meth)acrylate;
polyoxyalkylene glycol di(meth)acrylate, such as diethylene glycol di(meth)acrylate, dipropylene glycol di(meth)acrylate, triethylene glycol (meth)acrylate, tetraethylene glycol di(meth)acrylate, polyethylene glycol di(meth)acrylate, or neopentyl glycol di(meth)acrylate;
a vinyl monomer having two or more ethylenic unsaturated bonds in its molecule, such as divinylbenzene;
an unsaturated polyester prepolymer obtained from at least one polyvalent carboxylic acid including ethylenic unsaturated polycarboxylic acid and at least one diol; and
a vinyl ester prepolymer obtained by acrylic modification of an end of an epoxy group.

Among those described above, the monomer that can be copolymerized with methyl methacrylate is preferably at least one selected from the group consisting of the methacrylate esters and the acrylate esters. This enables polymerization of the polymeric composition to obtain a methyl methacrylate polymer with excellent balance of transparency, heat resistance, and moldability. The monomer that can be copolymerized with methyl methacrylate is more preferably an acrylate ester, and particularly preferably at least one selected from the group consisting of methyl acrylate, ethyl acrylate, and n-butyl acrylate.
One or two or more monomer(s) that can be copolymerized with methyl methacrylate may be used.
The amount of the monomer that can be copolymerized with methyl methacrylate contained in the polymeric composition is preferably from 0 to 50 parts by mass with respect to 100 parts by mass of the methyl methacrylate-containing composition. This enables obtaining a methyl methacrylate polymer with high transparency. The upper limit of the amount of the monomer that can be copolymerized with methyl methacrylate with respect to 100 parts by mass of the methyl methacrylate-containing composition is more preferably 40 parts by mass or less, and still more preferably 30 parts by mass or less.

(Other Additives)

Other additives preferably include a polymerization initiator. Other additives may also include, for example, a chain transfer agent, a mold release agent, a lubricant, a plasticizer, an antioxidant, an antistatic agent, a light stabilizer, an ultraviolet absorber, a flame retardant, a flame retardant promoter, a polymerization inhibitor, a filler, a pigment, a dye, a silane coupling agent, a leveling agent, an antifoam, and a fluorescent agent, as needed. One or two or more other additive(s) may be contained.
Examples of the polymerization initiator include the following:

an azo compound, such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2-2'-azobis(2-methylpropane), 1,1-azobis(cyclohexanecarbonitrile), and dimethyl-2,2'-azobisisobutyrate;
an organic peroxide, such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butyl peroxy-2-ethylhexanoate, t-butyl peroxyisobutyrate, t-butyl peroxybenzoate, t-hexyl peroxybenzoate, t-butyl peroxyisopropyl monocarbonate, t-butyl peroxy-3,5,5-trimethyl hexanoate, t-butyl peroxylaurate, t-butyl peroxyacetate, t-hexyl peroxyisopropyl monocarbonate, t-hexyl peroxy-2-ethyl hexanoate, t-amyl peroxy-2-ethyl hexanoate, 1,1,3,3-tetramethyl butyl peroxyethyl hexanoate, 1,1,2-trimethyl propyl peroxy-2-ethyl hexanoate, 1,1,3,3-tetramethyl butyl peroxyisopropyl monocarbonate, 1,1,2-trimethyl propyl peroxyisopropyl monocarbonate, 1,1,3,3-tetramethyl butyl peroxyisononanoate, 1,1,2-trimethyl propyl peroxyisononanoate, di-t-butyl peroxide, di-t-hexyl peroxide, lauroyl peroxide, and dilauroyl peroxide;
a persulfate compound, such as potassium persulfate; and
a redox polymerization initiator.

Among those described above, the polymerization initiator is preferably at least one selected from the group consisting of the azo compound and the organic peroxide from the viewpoint of storage stability and reactivity with methyl methacrylate.
The amount of the polymerization initiator used is preferably from 0.0001 to 1 part by mass with respect to 100 parts by mass of the total of the methyl methacrylate-containing compound and the monomer that can be copolymerized with methyl methacrylate.

<Method of Polymerizing Polymeric Composition>

Examples of the polymerization method include a bulk polymerization method, a solution polymerization method, an emulsion polymerization method, and a suspension polymerization method. From the viewpoints of environmental load due to the use of solvents and the like, and of transparency of the methyl methacrylate polymer to be obtained, a bulk polymerization method is preferable.
Specific means for the bulk polymerization method is not particularly limited, and a known casting polymerization method such as a cell casting method or a continuous casting method can be used for production.
The casting polymerization method is a method for obtaining methyl methacrylate polymer by injecting a polymeric composition into a mold composed of two inorganic glass plates or metal plates (for example, SUS plates) placed opposite each other at a predetermined distance with the periphery sealed with a gasket such as a soft resin tube, and allowing the composition to polymerize.
The mold for casting polymerization is not particularly limited, and known molds can be used. Examples of the mold for cell casting include those in which two plate-like bodies, such as inorganic glass plates, chromium-plated metal plates, and stainless steel plates, are placed opposite each other at a predetermined distance, and a gasket is placed around the periphery to allow the plate-like bodies and the gasket to form a sealed space. Examples of the mold for continuous casting include those in which a sealed space is formed by opposing faces of a pair of endless belts running in the same direction at the same speed, and gaskets running at the same speed as the endless belt on both sides of the endless belt.
The spacing of the void in a mold is adjusted as appropriate to obtain a resin sheet with a desired thickness, and is generally from 1 to 30 mm.
The polymerization temperature is preferably from 125 to 210°C. This enables achieving an appropriate polymerization rate. More preferably, the lower limit of the polymerization temperature is 130°C or more, and the upper

limit is 180°C or less. The polymerization time is not particularly limited, and can be, for example, from 0.5 to 24 hours.

EXAMPLES

The present invention will be described in detail with reference to Examples and Comparative Examples below, but is not limited to these Examples. Unless otherwise stated, the terms "%" and "ppm" in the examples and the comparative examples mean "% by weight" and "ppm by weight," respectively.

The water concentration contained in a methyl methacrylate reagent was determined by the Karl-Fischer method. The composition of the components of the methyl methacrylate-containing composition before being stored was determined based on the amounts of the raw materials added. Methyl methacrylate dimers and methyl pyruvate in the methyl methacrylate-containing composition after being stored were determined by an absolute calibration curve method using GC-MS. The measurement conditions for GC-MS are shown below.

Instrument: GC-MS measuring system (product name: QP-2010SE, manufactured by Shimadzu Corporation)

[Conditions for GC]

Column (product name: DB-WAX, manufactured by Agilent Technologies, Inc.);
Length: 60 m, Inner diameter: 0.32 mm, Film thickness: 1.00 um;
Injection volume: 1.0 μL;
Vaporizing chamber temperature: 210°C;
Column oven temperature: held at 35°C for 10 minutes, raised from 35°C to 150°C at 5 °C/min, held at 150°C for 17 minutes, raised from 150°C to 220°C at 5 °C/min, and held at 220°C for 6 minutes;
Carrier gas: helium;
Injection mode: split (split ratio: 50);
Control mode: constant linear velocity (25.0 cm/sec);
Pressure: 26.1 KPa;
Total flow: 52.5 mL/min;
Purge flow: 3.0 mL/min; and
Column flow: 0.97 mL/min.

[Conditions for MS]

Ionization method: EI (Electron Ionization);
Ion source temperature: 250°C;
Interface temperature: 250°C;
m/z detection range: 10 to 300; and
Detection time: 70 minutes.

Determination of the water concentration by the Karl-Fischer method is carried out using automated water measurement equipment (product name: AQV-2200, manufactured by Hiranuma Co., Ltd.).

(Example 1)

Using methyl isobutyrate as a component A, 0.0293 g of the component A was added to 10.0822 g of a methyl methacrylate reagent (water concentration: 240 ppm) to prepare a methyl methacrylate solution (A-1 solution). The concentration of the component A in the A-1 solution is shown in Table 1.

Using 2,4-dimethyl-6-t-butylphenol as a component B, 0.0228 g of the component B was added to 10.0026 g of a methyl methacrylate reagent (water concentration: 240 ppm) to prepare a methyl methacrylate solution (B-1 solution). The concentration of the component B in the B-1 solution is shown in Table 1.

Next, 0.1693 g of the A-1 solution and 0.2007 g of the B-1 solution were added to 39.9556 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored at 25°C for 14 days. The amounts of methyl methacrylate dimers and methyl pyruvate produced in the methyl methacrylate-containing composition after the

storage are shown in Table 2.

(Examples 2 to 6)

A-1 solutions were prepared in the same manner as in Example 1 except that a compound shown in Table 1 was used as a component A, and the amounts of the methyl methacrylate reagent and the component A were changed as shown in Table 1.
B-1 solutions were prepared in the same manner as in Example 1.
Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.
The obtained methyl methacrylate-containing compositions were stored as in Example 1. The amounts of methyl methacrylate dimers and methyl pyruvate produced in the methyl methacrylate-containing compositions after the storage are shown in Table 2.

(Examples 7 to 13)

A-1 solutions were prepared in the same manner as in Example 1 except that a compound shown in Table 1 was used as a component A, and the amounts of the methyl methacrylate reagent and the component A were changed as shown in Table 1.
B-1 solutions were prepared in the same manner as in Example 1 except that the amounts of the methyl methacrylate reagent and the component B were changed as shown in Table 1.
Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.
The obtained methyl methacrylate-containing compositions were stored as in Example 1. The amounts of methyl methacrylate dimers and methyl pyruvate produced in the methyl methacrylate-containing compositions after the storage are shown in Table 2.

(Examples 14 to 19)

A-1 solutions were prepared in the same manner as in Example 1.
B-1 solutions were prepared in the same manner as in Example 1 except that a compound shown in Table 1 was used as a component B, and the amounts of the methyl methacrylate reagent and the component B were changed as shown in Table 1.
Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.
The obtained methyl methacrylate-containing compositions were stored as in Example 1. The amounts of methyl methacrylate dimers and methyl pyruvate produced in the methyl methacrylate-containing compositions after the storage are shown in Table 2.

(Example 20)

An A-1 solution and a B-1 solution were prepared in the same manner as in Example 1.
Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 2.
The obtained methyl methacrylate-containing composition was stored as in Example 1. The amounts of methyl methacrylate dimers and methyl pyruvate produced in the methyl methacrylate-containing composition after the storage are shown in Table 2.

(Example 21)

A B-1 solution was prepared in the same manner as in Example 1.
Next, using methyl isobutyrate as a component A, 0.0437 g of a component A and 0.2052 g of a B-1 solution was added to 40.0012 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate produced in the methyl methacrylate-containing composition after the storage are shown in Table 2.

(Example 22)

A B-1 solution was prepared in the same manner as in Example 1 except that the amounts of the methyl methacrylate reagent and the component B were changed as shown in Table 1.

Next, a methyl methacrylate-containing composition was prepared in the same manner as in Example 21 except that the amounts of the methyl methacrylate reagent, the component A, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate produced in the methyl methacrylate-containing composition after the storage are shown in Table 2.

(Example 23)

An A-1 solution and a B-1 solution were prepared in the same manner as in Example 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate produced in the methyl methacrylate-containing composition after the storage are shown in Table 2.

(Examples 24 and 25)

A-1 solutions were prepared in the same manner as in Example 1.

B-1 solutions were prepared in the same manner as in Example 1 except that the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate produced in the methyl methacrylate-containing compositions after the storage are shown in Table 2.

(Comparative Example 1)

A B-1 solution was prepared in the same manner as in Example 1.

Next, 0.2213 g of the B-1 solution was added to 40.0273 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate produced in the methyl methacrylate-containing composition after the storage are shown in Table 2.

(Comparative Example 2)

An A-1 solution was prepared in the same manner as in Example 1.

Next, 0.2123 g of the A-1 solution was added to 40.0630 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate produced in the methyl methacrylate-containing composition after the storage are shown in Table 2.

(Comparative Example 3)

40.0000 g of a methyl methacrylate reagent (water concentration: 240 ppm) was used as a methyl methacrylate-containing composition and stored as in Example 1. The amounts of methyl methacrylate dimers and methyl pyruvate produced in the methyl methacrylate-containing composition after the storage are shown in Table 2.

(Comparative Example 4)

A B-1 solution was prepared in the same manner as in Example 1.
Using diacetyl as a component C, 0.0205 g of the component C was added to 9.9913 g of a methyl methacrylate reagent (water concentration: 240 ppm) to prepare a methyl methacrylate solution (C-1 solution). The concentration of the component C in the C-1 solution is shown in Table 1.
Next, 0.2151 g of the B-1 solution and 0.2069 g of the C-1 solution were added to 40.0218 g of a methyl methacrylate reagent (water concentration: 240 ppm) to prepare a methyl methacrylate-containing composition. The concentrations of the components in the methyl methacrylate-containing composition are shown in Table 2.
The obtained methyl methacrylate-containing composition was stored as in Example 1. The amounts of methyl methacrylate dimers and methyl pyruvate produced in the methyl methacrylate-containing composition after the storage are shown in Table 2.

(Comparative Example 5)

A B-1 solution was prepared in the same manner as in Example 1 except that the amounts of the methyl methacrylate reagent and the component B were changed as shown in Table 1.
Next, a methyl methacrylate-containing composition was prepared in the same manner as in Example 11 except that the amounts of the methyl methacrylate reagent, the component A, and the B-1 solution were changed as shown in Table 2. The concentrations of the components in the methyl methacrylate-containing composition are shown in Table 2.
The obtained methyl methacrylate-containing composition was stored as in Example 1. The amounts of methyl methacrylate dimers and methyl pyruvate produced in the methyl methacrylate-containing composition after the storage are shown in Table 2.

(Comparative Example 6)

An A-1 solution was prepared in the same manner as in Example 1.
A B-1 solution was prepared in the same manner as in Example 1 except that the amounts of the methyl methacrylate reagent and the component B were changed as shown in Table 1.
Next, 0.0804 g of the A-1 solution, 0.1147 g of the B-1 solution, and 0.3221 g of pure water were added to 20.0231 g of a methyl methacrylate reagent (water concentration: 240 ppm) to prepare a methyl methacrylate-containing composition. The concentrations of the components in the methyl methacrylate-containing composition are shown in Table 2.
The obtained methyl methacrylate-containing composition was stored as in Example 1. The amounts of methyl methacrylate dimers and methyl pyruvate produced in the methyl methacrylate-containing composition after the storage are shown in Table 2.

Table 1

| | A-1 solution | | | | B-1 solution | | | | C-1 solution | | | |
|---|---|---|---|---|---|---|---|---|---|---|---|---|
| | Amount of MMA reagent added [g] | Component A | | | Amount of MMA reagent added [g] | Component B | | | Amount of MMA reagent added [g] | Component C | | |
| | | Compound name | Added amount [g] | Concentration [ppm] | | Compound name | Added amount [g] | Concentration [ppm] | | Compound name | Added amount [g] | Concentration [ppm] |
| Example 1 | 10.0822 | Methyl isobutyrate | 0.0293 | 2898 | 10.0026 | 2,4-Dimethyl-6-t-butylphenol | 0.0228 | 2274 | - | - | - | - |
| Example 2 | 10.1504 | Methyl propionate | 0.0179 | 1760 | 10.0026 | 2,4-Dimethyl-6-t-butylphenol | 0.0228 | 2274 | - | - | - | - |
| Example 3 | 9.9998 | Isobutyl isobutyrate | 0.0210 | 2096 | 10.0026 | 2,4-Dimethyl-6-t-butylphenol | 0.0228 | 2274 | - | - | - | - |
| Example 4 | 9.9929 | Methyl isovalerate | 0.0251 | 2505 | 10.0026 | 2,4-Dimethyl-6-t-butylphenol | 0.0228 | 2274 | - | - | - | - |
| Example 5 | 10.0090 | Methyl 2-methylbutyrate | 0.0212 | 2114 | 10.0026 | 2,4-Dimethyl-6-t-butylphenol | 0.0228 | 2274 | - | - | - | - |
| Example 6 | 10.1755 | Isoamyl isobutyrate | 0.0227 | 2226 | 10.0026 | 2,4-Dimethyl-6-t-butylphenol | 0.0228 | 2274 | - | - | - | - |
| Example 7 | 10.0181 | Methyl lactate | 0.0208 | 2072 | 40.008 | 2,4-Dimethyl-6-t-butylphenol | 0.0828 | 2065 | - | - | - | - |
| Example 8 | 10.0299 | N,N-Dimethylglycine methyl | 0.0258 | 2566 | 40.008 | 2,4-Dimethyl-6-t-butylphenol | 0.0828 | 2065 | - | - | - | - |
| Example 9 | 10.0110 | Dimethyl malonate | 0.0516 | 5128 | 40.008 | 2,4-Dimethyl-6-t-butylphenol | 0.0828 | 2065 | - | - | - | - |
| Example 10 | 10.0162 | Methyl (methylthio) acetate | 0.0225 | 2241 | 40.008 | 2,4-Dimethyl-6-t-butylphenol | 0.0828 | 2065 | - | - | - | - |
| Example 11 | 10.0046 | Methyl 3-butenoate | 0.0231 | 2304 | 40.008 | 2,4-Dimethyl-6-t-butylphenol | 0.0828 | 2065 | - | - | - | - |
| Example 12 | 10.0039 | Methyl (R)-(-)-3-hydroxyisobutyrate | 0.0228 | 2274 | 40.008 | 2,4-Dimethyl-6-t-butylphenol | 0.0828 | 2065 | - | - | - | - |
| Example 13 | 10.0052 | Methyl cyclohexanecarboxylate | 0.0226 | 2254 | 40.008 | 2,4-Dimethyl-6-t-butylphenol | 0.0828 | 2065 | - | - | - | - |

(continued)

| | A-1 solution | | | | B-1 solution | | | | C-1 solution | | | |
|---|---|---|---|---|---|---|---|---|---|---|---|---|
| | Amount of MMA reagent added [g] | Component A | | | Amount of MMA reagent added [g] | Component B | | | Amount of MMA reagent added [g] | Component C | | |
| | | Compound name | Added amount [g] | Concentration [ppm] | | Compound name | Added amount [g] | Concentration [ppm] | | Compound name | Added amount [g] | Concentration [ppm] |
| Example 14 | 10.0822 | Methyl isobutyrate | 0.0293 | 2898 | 10.0051 | 4-Methoxyphenol | 0.0191 | 1905 | - | - | - | - |
| Example 15 | 10.0822 | Methyl isobutyrate | 0.0293 | 2898 | 10.0238 | Hydroquinone | 0.0233 | 2319 | - | - | - | - |
| Example 16 | 10.0822 | Methyl isobutyrate | 0.0293 | 2898 | 10.0204 | Phenothiazine | 0.0238 | 2370 | - | - | - | - |
| Example 17 | 10.0822 | Methyl isobutyrate | 0.0293 | 2898 | 10.0021 | 4-Hydroxy-2,2,6,6-tetramethylpiperidine-N-oxyl | 0.0202 | 2016 | - | - | - | - |
| Example 18 | 10.0822 | Methyl isobutyrate | 0.0293 | 2898 | 10.1244 | N,N-Diphenylamine | 0.0222 | 2188 | - | - | - | - |
| Example 19 | 10.0822 | Methyl isobutyrate | 0.0293 | 2898 | 10.0077 | N-Nitrosodiphenylamine | 0.0208 | 2074 | - | - | - | - |
| Example 20 | 10.0822 | Methyl isobutyrate | 0.0293 | 2898 | 10.0026 | 2,4-Dimethyl-6-t-butylphenol | 0.0228 | 2274 | - | - | - | - |
| Example 21 | - | - | - | - | 10.0026 | 2,4-Dimethyl-6-t-butylphenol | 0.0228 | 2274 | - | - | - | - |
| Example 22 | - | - | - | - | 40.0221 | 2,4-Dimethyl-6-t-butylphenol | 0.0829 | 2067 | - | - | - | - |
| Example 23 | 10.0822 | Methyl isobutyrate | 0.0293 | 2898 | 10.0026 | 2,4-Dimethyl-6-t-butylphenol | 0.0228 | 2274 | - | - | - | - |
| Example 24 | 10.0822 | Methyl isobutyrate | 0.0293 | 2898 | 40.0221 | 2,4-Dimethyl-6-t-butylphenol | 0.0829 | 2067 | - | - | - | - |
| Example 25 | 10.0822 | Methyl isobutyrate | 0.0293 | 2898 | 40.0221 | 2,4-Dimethyl-6-t-butylphenol | 0.0829 | 2067 | - | - | - | - |
| Comparative Example 1 | - | - | - | - | 10.0026 | 2,4-Dimethyl-6-t-butylphenol | 0.0228 | 2274 | - | - | - | - |
| Comparative Example 2 | 10.0822 | Methyl isobutyrate | 0.0293 | 2898 | - | - | - | - | - | - | - | - |

(continued)

| | A-1 solution | | | | B-1 solution | | | | C-1 solution | | | |
|---|---|---|---|---|---|---|---|---|---|---|---|---|
| | Amount of MMA reagent added [g] | Component A | | | Amount of MMA reagent added [g] | Component B | | | Amount of MMA reagent added [g] | Component C | | |
| | | Compound name | Added amount [g] | Concen tration [ppm] | | Compound name | Added amount [g] | Concen tration [ppm] | | Compou nd name | Added amount [g] | Concen tration [ppm] |
| Comparative Example 3 | - | - | - | - | - | - | - | - | - | - | - | - |
| Comparative Example 4 | - | - | - | - | 10.0026 | 2,4-Dimethyl-6-t-butylphenol | 0.0228 | 2274 | 9.9913 | Diacetyl | 0.0205 | 2048 |
| Comparative Example 5 | - | - | - | - | 40.0221 | 2,4-Dimethyl-6-t-butylphenol | 0.0829 | 2067 | - | - | - | - |
| Comparative Example 6 | 10.0822 | Methyl isobutyrate | 0.0293 | 2898 | 40.0221 | 2,4-Dimethyl-6-t-butylphenol | 0.0829 | 2067 | - | - | - | - |

Table 2

| | Added amount [g] | | | | | | MMA-containing composition | | | | | | | | Amount of MMA dimer produced [ppm] | Amount ofmethyl pyruvate produced [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 solution | B-1 solution | C-1 solution | Component A | Pure water | Comcentration [%] | Compound name | Concentr ation MA [μmol/L] | Compound name | Concentr ation MB [μmol/L] | Compou nd name | Concentr ation [μmol/L] | | | |
| Example 1 | 39.9556 | 0.1693 | 0.2007 | - | - | - | 99.97 | Methyl isobutyrate | 112 | 2,4-Dimethyl-6-t-butylphenol | 60 | - | - | 0.533 | 0 | 6 |
| Example 2 | 40.0055 | 0.2002 | 0.2031 | - | - | - | 99.97 | Methyl propionate | 93 | 2,4-Dimethyl-6-t-butylphenol | 61 | - | - | 0.648 | 13 | 5 |
| Example 3 | 40.0212 | 0.2019 | 0.1993 | - | - | - | 99.97 | Isobutyl isobutyrate | 69 | 2,4-Dimethyl-6-t-butyl phenol | 59 | - | - | 0.867 | 13 | 6 |
| Example 4 | 40.2492 | 0.2152 | 0.2092 | - | - | - | 99.97 | Methyl isovalerate | 108 | 2,4-Dimethyl-6-t-butylphenol | 62 | - | - | 0.575 | 15 | 9 |
| Example 5 | 40.0097 | 0.2210 | 0.1984 | - | - | - | 99.97 | Methyl 2-methylbutyrate | 94 | 2,4-Dimethyl-6-t-butylphenol | 59 | - | - | 0.629 | 0 | 8 |
| Example 6 | 40.1339 | 0.2061 | 0.2052 | - | - | - | 99.97 | Isoamyl isobutyrate | 67 | 2,4-Dimethyl-6-t-butylphenol | 61 | - | - | 0.903 | 14 | 7 |
| Example 7 | 10.0026 | 0.0530 | 0.0545 | - | - | - | 99.97 | Methyl lactate | 98 | 2,4-Dimethyl-6-t-butylphenol | 59 | - | - | 0.599 | 19 | 7 |
| Example 8 | 10.0045 | 0.0508 | 0.0519 | - | - | - | 99.97 | N,N-Dimethylglycine methyl | 104 | 2,4-Dimethyl-6-t-butylphenol | 56 | - | - | 0.540 | 23 | 5 |
| Example 9 | 10.0031 | 0.0525 | 0.0519 | - | - | - | 99.97 | Dimethyl malonate | 190 | 2,4-Dimethyl-6-t-butyl phenol | 56 | - | - | 0.295 | 17 | 9 |
| Example 10 | 10.0641 | 0.0517 | 0.0505 | - | - | - | 99.97 | Methyl (methylthio) acetate | 90 | 2,4-Dimethyl-6-t-butylphenol | 54 | - | - | 0.607 | 20 | 9 |
| Example 11 | 10.0029 | 0.0558 | 0.0507 | - | - | - | 99.97 | Methyl 3-butenoate | 120 | 2,4-Dimethyl-6-t-butylphenol | 55 | - | - | 0.457 | 17 | 11 |
| Example 12 | 10.0018 | 0.0505 | 0.0538 | - | - | - | 99.97 | Methyl (R)-(-)-3-hydroxyisobutyrate | 91 | 2,4-Dimethyl-6-t-butylphenol | 58 | - | - | 0.641 | 18 | 8 |

(continued)

| | Added amount [g] | | | | | | MMA-containing composition | | | | | | | | Amount of MMA dimer pro-duced [ppm] | Amount ofmethyl pyru-vate pro-duced [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 so-lution | B-1 so-lution | C-1 so-lution | Com-ponent A | Pure water | Com-cen tra-tion [%] | Compound name | Concen-tr ation MA [μmol/L] | Compound name | Concen-tr ation MB [μmol/L] | Com-pou nd name | Concen-tr ation [μmol/L] | | | |
| Example 13 | 10.006 2 | 0.0535 | 0.0575 | - | - | - | 99.97 | Methyl cyclohexanecarboxylate | 79 | 2,4-Dimethyl-6-t-butylphenol | 62 | - | - | 0.786 | 21 | 12 |
| Example 14 | 40.024 0 | 0.1351 | 0.2305 | - | - | - | 99.97 | Methyl isobutyrate | 90 | 4-Methoxyphenol | 83 | - | - | 0.923 | 0 | 6 |
| Example 15 | 40.008 3 | 0.1504 | 0.2028 | - | - | - | 99.97 | Methyl isobutyrate | 100 | Hydroquinone | 100 | - | - | 1.001 | 0 | 4 |
| Example 16 | 40.007 7 | 0.1609 | 0.1982 | - | - | - | 99.97 | Methyl isobutyrate | 107 | Phenothiazine | 55 | - | - | 0.516 | 11 | 5 |
| Example 17 | 10.002 2 | 0.0372 | 0.0522 | - | - | - | 99.97 | Methyl isobutyrate | 99 | 4-Hydroxy-2,2,6,6-tetramethylpiperidine-N-oxyl | 57 | | - | 0.579 | 0 | 11 |
| Example 18 | 10.000 7 | 0.0362 | 0.0515 | - | - | - | 99.97 | Methyl isobutyrate | 96 | N,N-Diphenylamine | 62 | - | - | 0.648 | 7 | 0 |
| Example 19 | 10.053 3 | 0.0361 | 0.0578 | - | - | - | 99.97 | Methyl isobutyrate | 95 | N-Nitrosodiphenylamine | 56 | - | - | 0.590 | 7 | 0 |
| Example 20 | 40.033 0 | 1.4447 | 0.2076 | - | - | - | 99.96 | Methyl isobutyrate | 928 | 2,4-Dimethyl-6-t-butylphenol | 60 | - | - | 0.065 | 0 | 0 |
| Example 21 | 40.001 2 | - | 0.2052 | - | 0.0437 | - | 99.87 | Methyl isobutyrate | 10035 | 2,4-Dimethyl-6-t-butylphenol | 61 | - | - | 0.006 | 16 | 7 |
| Example 22 | 10.006 6 | - | 0.0559 | - | 0.0808 | - | 99.18 | Methyl isobutyrate | 73629 | 2,4-Dimethyl-6-t-butylphenol | 60 | - | - | 0.001 | 18 | 8 |
| Example 23 | 39.997 3 | 0.1432 | 2.1248 | - | - | - | 99.96 | Methyl isobutyrate | 91 | 2,4-Dimethyl-6-t-butylphenol | 605 | - | - | 6.671 | 15 | 4 |

(continued)

| | Added amount [g] | | | | | | MMA-containing composition | | | | | | | | Amount of MMA dimer produced [ppm] | Amount of methyl pyruvate produced [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 solution | B-1 solution | C-1 solution | Component A | Pure water | Concentration [%] | Compound name | Concentration MA [μmol/L] | Compound name | Concentration MB [μmol/L] | Compound name | Concentration [μmol/L] | | | |
| Example 24 | 7.5170 | 0.0409 | 2.5003 | - | - | - | 99.92 | Methyl isobutyrate | 109 | 2,4-Dimethyl-6-t-butylphenol | 2721 | - | - | 24.982 | 21 | 0 |
| Example 25 | 3.8356 | 0.0383 | 6.2948 | - | - | - | 99.85 | Methyl isobutyrate | 101 | 2,4-Dimethyl-6-t-butylphenol | 6776 | - | - | 67.164 | 21 | 0 |
| Comparative Example 1 | 40.0273 | - | 0.2213 | - | - | - | 99.97 | - | - | 2,4-Dimethyl-6-t-butylphenol | 66 | - | - | - | 29 | 2 |
| Comparative Example 2 | 40.0630 | 0.2123 | - | - | - | - | 99.97 | Methyl isobutyrate | 141 | - | - | - | - | - | 0 | 32 |
| Comparative Example 3 | 40.0000 | - | - | | - | - | 99.98 | - | - | - | - | - | - | - | 0 | 25 |
| Comparative Example 4 | 40.0218 | - | 0.2151 | 0.2069 | - | - | 99.97 | - | - | 2,4-Dimethyl-6-t-butylphenol | 64 | Diacetyl | 115 | - | 0 | 1056 |
| Comparative Example 5 | 19.9826 | - | 0.1002 | - | 0.3071 | - | 98.47 | Methyl isobutyrate | 139214 | 2,4-Dimethyl-6-t-butylphenol | 54 | - | - | 0.000 | 29 | 18 |
| Comparative Example 6 | 20.0231 | 0.0804 | 0.1147 | - | - | 0.3221 | 98.41 | Methyl isobutyrate | 105 | 2,4-Dimethyl-6-t-butylphenol | 61 | - | - | 0.583 | 32 | 2 |

As shown in Tables 1 and 2, Examples 1 to 25 in which the methyl methacrylate-containing composition contains defined components A and B show reduction of both production of methyl methacrylate dimers and methyl pyruvate in the methyl methacrylate compositions after storage, and can be said to have high quality stability during storage.

The polymeric composition comprising each methyl methacrylate-containing composition obtained in each Example can be polymerized to obtain a methyl methacrylate polymer.

INDUSTRIAL APPLICABILITY

According to the present invention, methyl methacrylate-containing compositions that can be used, for example, as raw materials for acrylic resins can be stored stably for a long period of time, which is industrially useful.

## Claims

1. A methyl methacrylate-containing composition, comprising:

    methyl methacrylate;
    an ester compound having an alpha-hydrogen represented by the following Formula (1) (component A); and
    a polymerization inhibitor (component B),
    wherein the concentration of methyl methacrylate is from 99 to 99.99% by mass,

    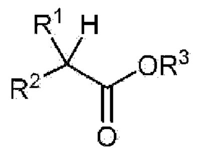 (1)

    wherein, in Formula (1) above,

    $R^1$ and $R^2$ each independently represent a hydrogen atom, an alkyl group, an alkenyl group, a hydroxy group, an alkoxy group, an amino group, a monovalent group containing a carbonyl group, or an alkylthio group,
    $R^3$ represents an alkyl group or an aryl group, and
    $R^1$ and $R^2$, $R^2$ and $R^3$, and $R^3$ and $R^1$ each may be linked to each other to form a ring.

2. The methyl methacrylate-containing composition according to claim 1, wherein when the concentration of the component A is MA (umol/L) and the concentration of the component B is MB (μmol/L), MB/MA is from 0.005 to 7.

3. The methyl methacrylate-containing composition according to claim 1 or 2, wherein the MB/MA is from 0.05 to 5.

4. The methyl methacrylate-containing composition according to any one of claims 1 to 3, wherein the MA is from 1 to 50,000 (μmol/L).

5. The methyl methacrylate-containing composition according to any one of claims 1 to 4, wherein the MA is from 10 to 30,000 (μmol/L).

6. The methyl methacrylate-containing composition according to any one of claims 1 to 5, wherein the MB is from 1 to 5,000 (μmol/L).

7. The methyl methacrylate-containing composition according to any one of claims 1 to 6, wherein the MB is from 10 to 1,000 (μmol/L).

8. The methyl methacrylate-containing composition according to any one of claims 1 to 7, wherein the molecular weight of the component A is 1,000 or less.

9. The methyl methacrylate-containing composition according to any one of claims 1 to 8, wherein the component B is at least one polymerization inhibitor selected from the group consisting of a phenol compound, a quinone compound, a nitrobenzene compound, an N-oxyl compound, an amine compound, a phosphorus-containing compound,

a sulfur-containing compound, an iron-containing compound, a copper-containing compound, and a manganese-containing compounds.

**10.** The methyl methacrylate-containing composition according to any one of claims 1 to 9, wherein the component B is at least one polymerization inhibitor selected from the group consisting of a phenol compound and a sulfur-containing compound.

**11.** The methyl methacrylate-containing composition according to any one of claims 1 to 10, wherein the component B is at least one polymerization inhibitor selected from the group consisting of hydroquinone, 4-methoxyphenol, 2,4-dimethyl-6-t-butylphenol, 2,6-di-t-butyl-4-methylphenol, and phenothiazine.

**12.** The methyl methacrylate-containing composition according to any one of claims 1 to 11, wherein the concentration of methyl methacrylate is from 99.8 to 99.99% by mass.

**13.** The methyl methacrylate-containing composition according to any one of claims 1 to 12, wherein the composition does not contain diacetyl, or the concentration of diacetyl contained is 55 (umol/L) or less.

**14.** The methyl methacrylate-containing composition according to any one of claims 1 to 13, wherein in Formula (1),

$R^1$ and $R^2$ are each independently a hydrogen atom, a $C_{1-5}$ alkyl group, a hydroxy group, a $C_{1-6}$ alkoxy group, an amino group, a monovalent group containing a carbonyl group, or a $C_{1-5}$ alkylthio group, and
$R^3$ is a $C_{1-5}$ alkyl group or a $C_{5-12}$ aryl group.

**15.** The methyl methacrylate-containing composition according to any one of claims 1 to 14, wherein in Formula (1),

$R^1$ and $R^2$ are each independently a hydrogen atom or a $C_{1-5}$ alkyl group, and
$R^3$ is a $C_{1-5}$ alkyl group.

**16.** The methyl methacrylate-containing composition according to any one of claims 1 to 15, wherein in Formula (1),

$R^1$ and $R^2$ are each independently a hydrogen atom, a methyl group, an ethyl group, an n-propyl group, or an isopropyl group, and
$R^3$ is a methyl group.

**17.** A method of producing a methyl methacrylate polymer, comprising a step of polymerizing a polymeric composition comprising a methyl methacrylate-containing composition according to any one of claims 1 to 16.

**18.** The method of producing a methyl methacrylate polymer according to claim 17, wherein the polymeric composition comprises a monomer that can be copolymerized with methyl methacrylate.

## INTERNATIONAL SEARCH REPORT

International application No.
**PCT/JP2022/016404**

**A. CLASSIFICATION OF SUBJECT MATTER**

***C07C 39/06***(2006.01)i; ***C08F 110/14***(2006.01)i; ***C08F 2/40***(2006.01)i; ***C07C 69/24***(2006.01)i; ***C07C 69/54***(2006.01)i
FI: C08F110/14; C07C69/24; C07C39/06; C07C69/54 Z; C08F2/40

According to International Patent Classification (IPC) or to both national classification and IPC

**B. FIELDS SEARCHED**

Minimum documentation searched (classification system followed by classification symbols)

C07C39/06; C08F110/14; C08F2/40; C07C69/24; C07C69/54

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-533624 A (BASF AKTIENGESELLSCHAFT) 22 November 2007 (2007-11-22) claims, paragraph [0102] | 1-14, 17-18 |
| A | | 15-16 |
| A | JP 2003-119205 A (ASAHI DENKA KOGYO K.K.) 23 April 2003 (2003-04-23) claims, examples | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 June 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/016404**

| Patent document cited in search report | Publication date (day/month/year) | Patent family member(s) | Publication date (day/month/year) |
|---|---|---|---|
| JP 2007-533624 A | 22 November 2007 | US 2006/0287548 A1<br>claims, paragraph [0099]<br>WO 2005/030907 A1<br>EP 1518916 A1<br>CN 1878852 A | |
| JP 2003-119205 A | 23 April 2003 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004155757 A
- JP 2005502695 A
- JP H10504553 A
- JP 2002533309 A
- JP 2002513034 A


### Non-patent literature cited in the description


- Development of Catalyst for Producing Methyl Methacrylate. **TORU KURODA.** Catalysts & Catalysis. Catalysis Society of Japan, 2003, vol. 45, 366-371
- On the Functions of Polymerization Inhibitors. **TAKAYUKI OTSU.** Synthetic Organic Chemistry. The Society of Synthetic Organic Chemistry, 1975, vol. 33, 634-640